# EUROPEAN PATENT APPLICATION

(11) **EP 4 169 548 A1**
(43) Date of publication of application: **26.04.2023**
(21) Application number: 21204545.4
(22) Date of filing: 25.10.2021
(51) Int. Cl.: A61M 5/142

(54) **POWER SUPPLY MODULE FOR A MOBILE DRUG DELIVERY DEVICE**

(71) Applicant: Ypsomed AG, 3401 Burgdorf (CH)
(72) Inventor: Rihs, Jonas, 2552 Orpund (CH); Friedli, Mirco, 3303 Jegenstorf (CH)
(74) Representative: Meier Obertüfer, Jürg

(57) **Abstract**

The present invention describes a mobile or wearable drug delivery device with a battery, a control circuitry and an improved power supply module. The power supply module includes a first power supply circuitry configured to providing electric current to the control circuitry in an active drug delivery mode, and a second power supply circuitry configured to bypass the first power supply circuitry and to provide electric current to the control circuitry in a storage mode. The control circuitry includes a power select line configured to switch the second power supply module from storage mode to active mode. The second power supply circuitry may comprise a diode and a FET transistor. The control circuitry may use an activation signal from a start circuitry to trigger a change from storage mode to active mode.

## Description

### FIELD OF THE INVENTION

The present invention relates to a drug delivery device with a battery, an electric motor, and to a power supply module for such a device.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Drug delivery devices include injection devices that are removed from the site of application after each medication event or drug delivery process, as well as infusion devices with a cannula or needle that remains in the skin of the patient for a prolonged period of time. By way of example, diabetes may be treated by administration of insulin by the patients themselves with the help of multi-variabledose insulin injection pens or infusion pumps. Alternatively, patch injectors, wearable injectors or wearable pumps are patched or adhered to the skin of the patient.

Common to all devices for subcutaneous drug delivery is a reservoir to store the fluid medicament, and a fluid path to bring the drug out of the device and into the subcutaneous tissue of a patient. In a majority of injecting or infusion devices the reservoir has a plunger which is mechanically advanced by an actuation assembly - in this case usually a plunger rod - to drive the fluid out of the reservoir into the fluid path and towards the patient. Alternatively, a pump mechanism such as a peristaltic, membrane or piston pump may be used to transport the fluid and effectuate the drug delivery, with a motor-driven actuation assembly generating the mechanical movement.

In many drug delivery devices, an electric motor is used as a driving means. For example, mobile devices such as insulin pumps or patch injectors typically use an electric battery to power the pump mechanism or injection apparatus. In such an arrangement, an electronic circuitry with a microprocessor controls an electric power supply module to provide the electric power at various voltage levels required to operate the drug delivery device. The electric power is sourced from a battery, typically a rechargeable battery built into the device. Devices with a replaceable battery often have a built-in rechargeable battery as a backup to ensure fail-safe operation. If a battery is assembled or inserted in a device at the time of manufacturing, for example for a ready-to-use device such as a pre-filled wearable bolus injector, the device will spend a considerable amount of time in a storage mode before being activated for drug delivery. In drug delivery mode, a number of supply currents are needed at different voltage levels for driving the motor, supervising the drug delivery, providing a user interface or wireless communication. For drug delivery devices with built-in or pre-inserted battery it is desirable to achieve a maximum storage time with a given battery while still ensuring that sufficient power is available for drug delivery. This asks for an electric power supply module with a minimal current in storage mode and sufficient current at specified voltages in drug delivery mode. With a battery of a given capacity, the power consumption in storage mode defines the shelf life during which a device may be stored after manufacturing until activation and use. A longer shelf life is a major advantage both for rechargeable drug delivery devices, where users need to charge the device before use, and for single use drug delivery devices, which have to be disposed of unused when the battery is too low for correct application.

It is an object of the current invention to provide a mobile or wearable drug delivery device with an increased shelf life while ensuring sufficient battery power for safe and reliable delivery of a medicament.

A number of existing solutions use a DC-DC converter to provide the voltage levels for drug delivery in active mode, and an electromechanical switch to cut off the battery supply in storage mode. A simple form of an electromechanical switch is a sheet of plastic removably mounted at one of the battery poles. The electromechanical switch cuts off the battery completely from the electronic circuitry and all the electric components connected thereto. Hence, the switch reduces power consumption in storage mode to zero. However, operating the electromechanical switch requires an extra manual handling step by the user. This can be a considerable problem for drug delivery devices, where minimum complexity of use is essential to minimise the probability of use errors. It is therefore an object of the current invention to provide a mobile or wearable drug delivery device with an increased shelf life and no extra manual operating step to activate the electric power.

DE10041845A1 describes a mobile drug delivery device, more specifically a iontophoretic patch device, where the electromechanical switch is integrated into the adhesive patch of the device. The patch device has a battery and a power supply circuit including a FET transistor switched by a microprocessor. In storage mode, the microprocessor power is cut off from the battery by the electromechanical switch. The microprocessor power is activated by the user when removing the liner of the patch adhesive. After activation of the microprocessor power, a number of options is described how the microprocessor may decide to activate the FET transistor, to switch on the power for drug delivery, and to enter drug delivery mode.

An alternative to the electromechanical switch is an electronic switch operated by a control circuitry, typically with a microprocessor, where the activation of the device can be controlled by software based on input signals from any kind of sensors. This obviates the unwanted extra handling step but makes power management more difficult. In such a configuration the microprocessor and the sensors need to be powered prior to switching the main power supply - either during all of the time in storage mode, or at least during activation. Hence, the power supply circuit typically includes two circuitries, a first power supply circuitry PWR1 with a DCDC converter providing power to the main components only in drug delivery mode, and a second power supply circuitry PWR2 providing power VUC to the control circuitry with the microprocessor and as little periphery as possible. A second battery may also be introduced, as for example disclosed in WO2017/181325.

A typical power supply module for a drug delivery device has a main power supply circuitry PWR1 and a bypass power supply circuitry PWR2 bypassing PWR1 and combining the output of PWR1 with the output of the battery to supply the control circuitry. PWR2 works as a power selector or combiner, drawing electric current from PWR1 in delivery mode, and drawing electric current from the battery when PWR1 is switched off in storage mode. To achieve the object of the current invention with such a power supply module, PWR1 needs to provide optimum output current at stable output voltage levels in delivery mode, while PWR2 needs to combine its two input power sources to an output power connection to the control circuitry with a minimum current in storage mode, and with an ideally loss-free passage from the output of PWR1 in delivery mode. The latter is of particular importance for mobile or wearable devices where the control circuitry typically includes or powers components with a requirement for high current, such as for example a wireless communication unit, high current hall-sensors for motor supervision or other burst-driven components.

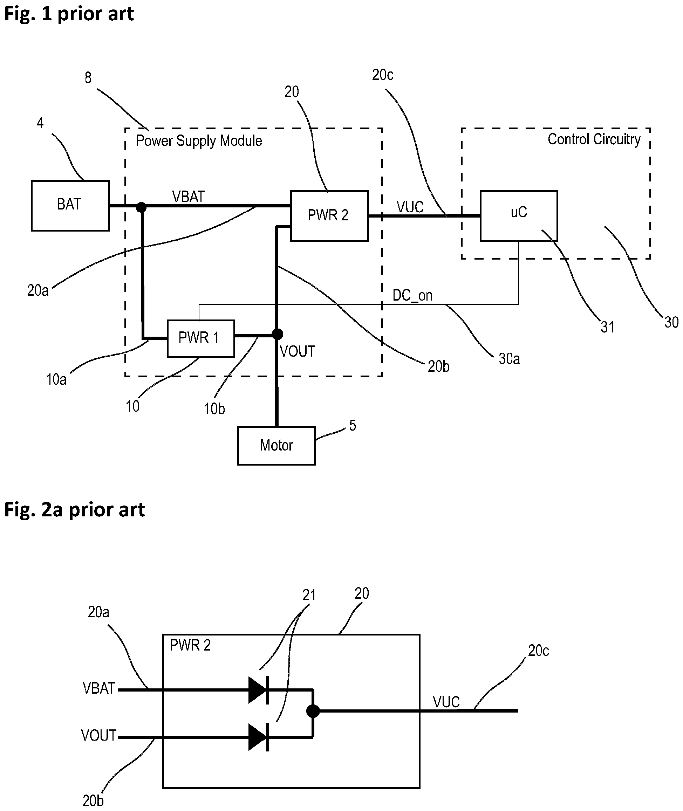

Fig. 1 shows the block diagram of such a power supply module suitable for mobile or wearable drug delivery devices, with a main power supply circuitry PWR1 and a bypass power supply circuitry PWR2 specifically for the control circuitry with the microprocessor. Thick lines labelled VBAT, VOUT and VUC depict power lines, thin lines such as DC_on depict control lines. The control circuitry typically includes an electronic control line DC_on to switch the output of the main power supply circuitry PWR1 off and control the activity of the motor and other components.

Various power supply modules exist adapting the first or only power supply circuitry PWR1 to the characteristics of the motor, as for example in US2003/0049135Alwhere the motor is a Solenoid actuator, or in US7927326B2 where the power supply circuitry includes a capacitive energy storage for residual energy recovery from the motor. While improving power consumption during drug delivery, these solutions do not address the power consumption in storage mode.

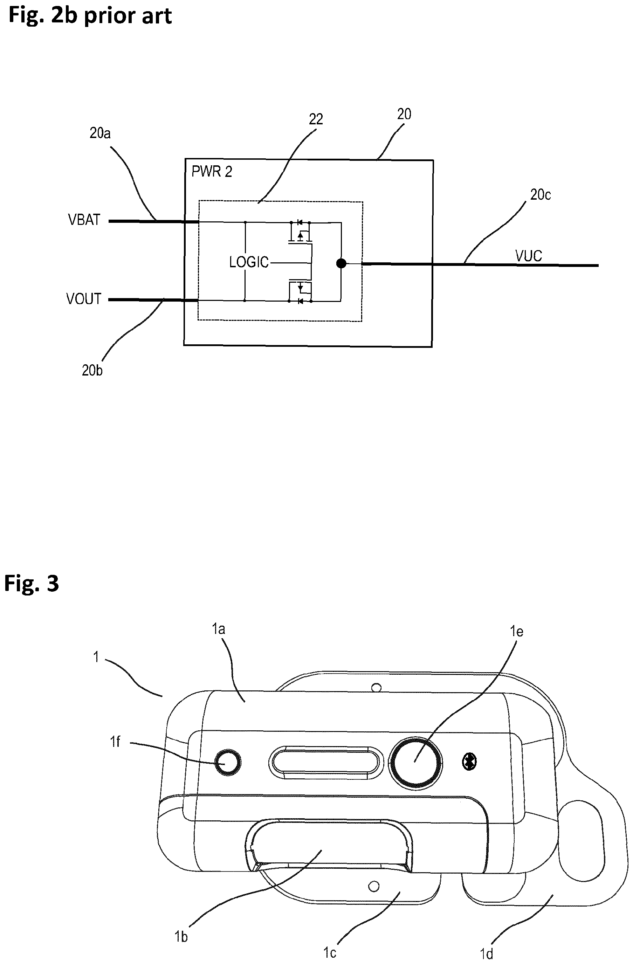

Fig. 2 illustrates two known examples of bypass power supply circuitries PWR2. Fig. 2a shows an alternative with two diodes 20 to prevent the current from flowing backwards from one source to an other. The diodes 21 block the reverse current almost completely, but at the price of a voltage drop of typically 0.3V to 0.7V in forward direction. This means that for the control circuitry, the output current of PWR1 is only available at a lower voltage, which may constitute a considerable loss.

In Fig. 2b, the two diodes are replaced by a FET transistor and an "ideal diode", a circuitry realising a nearly loss-free connection from an input connection to the output connection when switched ON and a nearly leakage-free connection from an input connection to the output connection when switched OFF. Electronic components are commercially available realising this behaviour and provide the functionality of the bypass circuitry PWR2 with two FET transistors. An internal logic circuitry of the ideal diode components drives the gates of the FET transistors to switch on and off the two power sources. Such a component may achieve near zero voltage drop for power from the output of PWR1 during drug delivery, but the internal logic needs to be powered permanently, resulting in a leakage current in storage mode which is disadvantageous for mobile or wearable devices.

The current invention increases the shelf life of a mobile or wearable drug delivery device while ensuring sufficient battery power for safe and reliable delivery of a medicament by providing an improved power supply module, where the bypass power supply circuitry PWR2 is controlled by the microprocessor and optimised to provide a minimum current in storage mode, and to provide a substantially loss-free power transmission from PWR1 for drug delivery when the device is activated.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The term "power supply module" is used in this document to include the entirety of power supply circuitry of the device. This does not imply or suggest that the power supply module is necessarily a distinct physical entity. The power supply module may be realised as a distinct physical component or assembly, may be integrated into a battery pack or may be realised as a circuitry distributed over any other assemblies in the drug delivery device.

### DESCRIPTION OF THE INVENTION

The improvement aims at realising a maximum time in the storage mode while still ensuring sufficient energy for drug delivery in the active mode. A preferred application of the improved power supply module may for example be for a body-worn drug delivery device such as a patch injector or patch pump, shipped ready to use with a factory mounted battery, where long storage time and optimum battery usage are most advantageous.

This objective is achieved by a mobile or wearable drug delivery device 1 comprising a battery, an electric motor for actuating a pump assembly to convey a medical fluid from a reservoir to a fluid output; an electronic control circuitry comprising a microprocessor configured to control the operation of the drug delivery device; an electric power supply module with a first power supply circuitry PWR1 having a converter input power line connected to the battery, a DC-DC converter and a converter output power line connected to the motor; and with a second power supply circuitry having a first input power line connected to the battery, a second input power line connected to the converter output power line, and a control output power line connected to the control circuitry; configured to conduct electric current from the first input power line or the second input power line to the control output power line. The drug delivery device is improved by introducing a power select line in the control circuitry, by connecting said power select line to the second power supply circuitry PWR2, by configuring the power select line to adopt at least two different logic states, and by configuring the second power supply circuitry PWR2 to switch on and off the electrical current from one of the input power lines to the control output power line according to the logic state of the power select line. This arrangement for an improved power supply module with a power select line allows the control circuitry to switch the power supply module from a storage mode, where the control circuitry is substantially supplied with electric current from the first input power line, to an active mode, where the control circuitry is substantially supplied with electric current from the second input power line, by switching the power select line from a first logic state to a second logic state.

More specifically, the second power supply circuitry PWR2 may comprise a diode in the electrical path from the first input power line, and a FET transistor in the electrical path from the second input power line, whereby the gate input of the FET transistor is connected to the power select line.

The mobile or wearable drug delivery device may further comprise a start circuitry with a device activation signal line, configured to generate a device activation signal for the control circuitry as a trigger signal to change the mode of the power supply module from storage mode to active mode. One embodiment of a start circuitry includes a mechanical start button, other embodiments may include any kind of optic, acoustic, electric or thermal sensor to detect if the device is being prepared for use or applied to the body of a patient.

More details about the improved power supply module are specified in corresponding dependent claims.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a general concept of a power supply module for a drug delivery device with a bypass power supply circuitry PWR2 for the control circuitry;
- Fig. 2a: depicts a first straightforward example of a bypass power supply circuitry PWR2 connecting two pathways using two diodes;
- Fig. 2b: depicts a known alternative bypass power supply circuitry PWR2 connecting two pathways using an ideal diode component and a FET transistor;
- Fig.3: depicts a wearable drug delivery device or patch injector;
- Fig.4a: depicts a cut through the patch injector of Fig. 3;
- Fig.4b: depicts the patch injector of Fig. 3 with the housing removed;
- Fig.5: depicts the concept of an improved power supply module for a drug delivery device according to the present invention, with two power supply control signals;
- Fig. 6: depicts a power supply circuitry connecting two input power leads using a diode and a FET transistor;

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

At the core of the present invention is a novel power supply module for use in a mobile or wearable drug delivery device. Fig. 3 shows a wearable patch injector 1 as a first preferred embodiment of a drug delivery device improved with such a power supply module. While the invention is described using the embodiment of a patch injector, it is evident that the same improvement may be implemented in any other drug delivery device such as an infusion pump or a patch pump with an adhesive patch. All improved drug delivery devices may consist of one single assembly with a complete housing 1a, or a modular arrangement of components attached together for operation. The housing 1a of the patch injector 1 has an opening or window 1b to allow sight of the reservoir 2 inside. The reservoir 2 may be pre-filled during manufacturing or filled manually using a syringe through a fill port If in the housing 1a when preparing the injector for drug delivery. The housing 1a is mounted on an adhesive patch 1c to attach the injector to the body of a patient for application. The adhesive patch 1c typically has a protective liner Id at the bottom surface towards the patient. To apply the patch injector 1, the user removes the protective liner Id from the adhesive patch 1c, attaches the patch injector 1 to the body of a patient and presses a start button 1e.

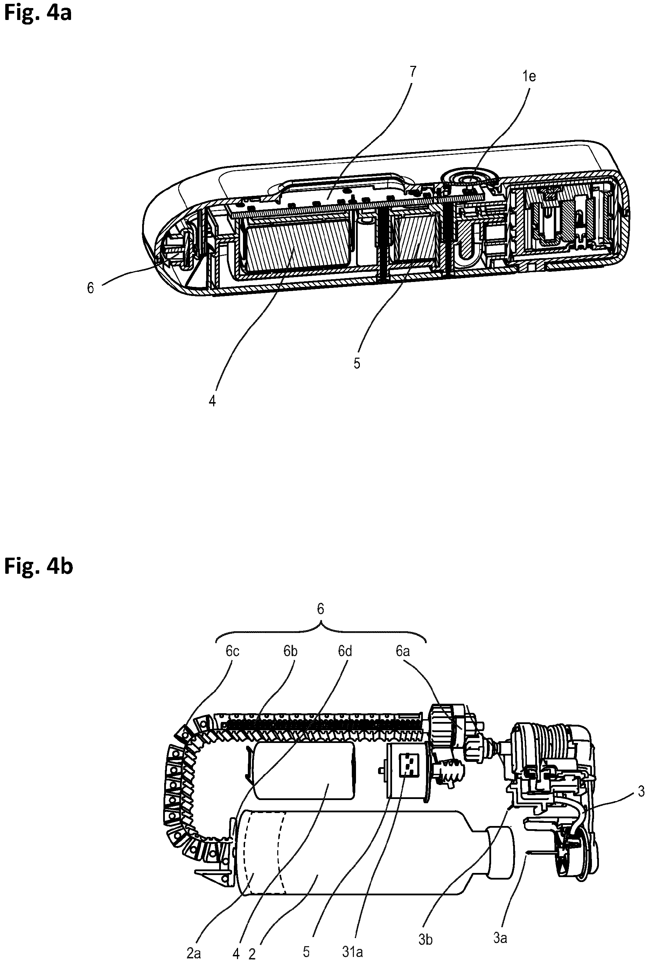

Fig. 4 illustrates a number of main components of the patch injector 1 of Fig. 3. Fig. 4a is a cut through the patch injector 1 showing the battery 4, a printed circuit board PCB 7 carrying electronic components and circuitry for example for the power supply module 8, for the user interface, for electronic communication, for the start circuitry 40 and for the electronic control circuitry 30. Fig. 4b shows the patch injector of Fig. 3 with the housing 1a and other parts removed to illustrate the mechanical implementation of the injection in more detail. In this embodiment, the reservoir 2 is pre-filled with a liquid medicine during manufacturing. For drug delivery, the fluid transport assembly 3 is brought into fluid connection with the reservoir 2. In a preferred embodiment, said fluid connection is established by pushing a piercing needle 3a or other form of fluid input through a septum (not shown) into the reservoir 2. This step may be part of the manufacturing process, or may be performed when the injector is being prepared for drug delivery. The fluid output 3b of the fluid transport assembly 3 may be the tip of a delivery needle 3b, as preferred for a patch pump or patch injector and illustrated in Fig. 4. Alternative embodiments may have a separate assembly with the delivery needle 3b, for example an infusion set as known for mobile infusion pumps. For application of the drug, both ends of the fluid transport assembly 3 are connected - the fluid input 3a with the reservoir 2, and the fluid output 3b with the body of the patient. Both connections may require manual action by the user, or may be established using an automated mechanical movement. In the preferred embodiment of the patch injector from Fig.3, the piercing needle 3a is connected with the reservoir 2 during manufacturing, and the delivery needle 3b is inserted into the body of the patient using an insertion mechanism 3c.

When activated by the control circuitry 30, the motor 5 actuates the pump assembly 6 and moves the plunger 2a in the reservoir 2 towards the fluid transport assembly 3. Fig. 4b shows the drive train in more detail, with a gear box assembly 6a transferring the rotation of the motor to a threaded rod 6b, pushing the segmented rod 6c along its path inside the housing (see Fig. 4a), generating a moving force to the plunger moving head 6d and from there to the plunger 2a. Any other kind of pump assembly 6 may be used, where the motor 5 induces a movement of a medical fluid out of a reservoir 2.

With the reservoir filled with medical fluid, and the fluid transport assembly 3 properly connected to the reservoir 2 and to the body of the patient - as described above - the movement of the plunger 2a directly results in application of medical fluid to the body of the patient. The electronic control circuitry 30 can effectuate and supervise the drug delivery as intended and controlled by the user.

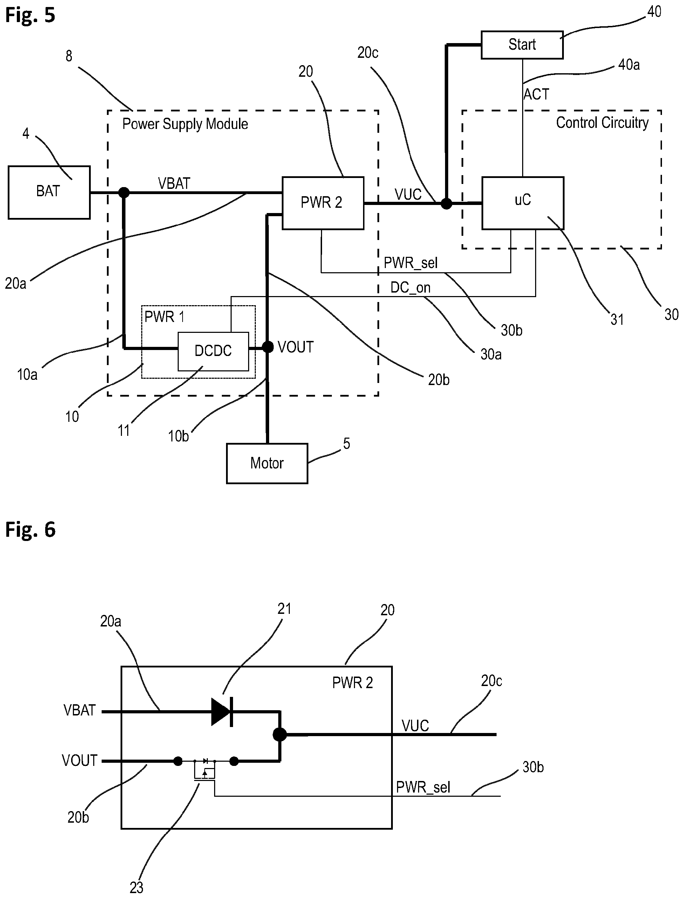

To achieve a maximum time in the storage mode while still ensuring sufficient energy for drug delivery in the active mode, a novel improved power supply module is introduced. Its structure is outlined in the block diagram of Fig. 5. Thick lines labelled VBAT, VOUT and VUC depict power lines, thin lines such as DC_on, PWR_sel and ACT represent control lines. The improved power supply module 8 has a first power supply circuitry 10 corresponding to the main power supply circuitry PWR1 of Fig. 1, and a second power supply circuitry 20 corresponding to the bypass power supply circuitry PWR2 of Fig.1. The first power supply circuitry 10 includes a DC-DC converter 11 connected to the battery 4 via a converter input power line 10a and provides a stable output voltage at the converter output power line 10b when activated by the converter output activation line 30a from the control circuitry 30. The control circuitry 30 enables the converter output power during active mode to power the motor and other components used in active mode only. The DC-DC converter 11 ensures that the motor and other components used in active mode have sufficient power even if the battery is nearly empty and the voltage at the converter input power line 20a has dropped below the voltage required by the motor. The second power supply circuitry 20 provides electric power to the control circuitry 30 at all times. It is a key aspect of the present invention that the second power supply circuitry 20 is controlled by the control circuitry 30, preferably by an output pin of the microprocessor 31 via a power select line 30b. In a preferred embodiment, the power select line 30b is a digital logic output of the microprocessor 31 with standard digital logic states of "0", "1", and "Z". The logic state of the power select line 30b is used to control the mode of the power supply module. A logic "0" may stand for storage mode and a logic "1" for active mode, or vice versa. If the power select line is implemented as an analog output of the microprocessor 31, two arbitrary output voltage levels may be assigned inside the analog output voltage range of, for example, 0V to 3V, to the storage mode and to the active mode, respectively. A voltage difference of at least 0.1V between the voltage levels will ensure that the second power supply circuitry 20 can use the power select line for robust detection of the logic state and of the corresponding mode to adopt. Other forms of electric signals such as pulse width modulation PWM may be used to feed the power select line and control the mode of the power supply module. More than two modes may be implemented for the power supply module by defining a multitude of logic states for the power select line, or by using a multitude of power select lines.

The introduction of a microprocessor-controlled power select line 30b for the purpose of selecting the source of the same power line the microprocessor 31 is powered from opens up a large range of possibilities to control the power supply module 8, to define operation modes for the power supply module 8, and to tailor the properties of the power supply module 8 to specific requirements of the control circuitry 30 in different operating modes.

The implementation of the second power supply circuitry 20 is another key aspect of the present invention. A preferred embodiment of the second power supply circuitry 20 is further illustrated in Fig. 6. A diode 21 is mounted at a first input power line 20a and a FET transistor 23 is mounted at a second input power line 20b. According to the concept of Fig. 5, the first input power line 20a is connected to the battery 4, and the second input power line 20b is connected to the converter output power line 10b. Both input branches of the second power supply circuitry 20 are jointly connected to a control output power line 20c to feed the control circuitry 30. The power select line 30b is connected to the gate of the FET transistor 23. The voltage level - and hence, the logic state - of the power select line 23 switches the current at the output of the FET transistor on and off. To put the power supply module 8 in storage mode, the microprocessor 31 defines the logic state of the power select line 30b to switch off the FET transistor. The FET transistor 23 can be optimised for low leakage current when switched off, leaving almost no current flowing from the second input power line 20b to the control output power line 20c in storage mode. Meanwhile, electric current may still flow from the first input power line 20a to the control output power line 20c. Even in storage mode, the control circuitry remains powered up and ready to control actions such as, for example, a periodical scanning of a start button 1e, a sensor or some other kind of start circuitry 40, temporarily drawing a current in the range of 1uA to 1mA. However, it is not optimal to use the current from the first input power line 20a in active mode where activities require higher current, because the diode 21 will typically cause a voltage drop of 0.3V to 0.7V. Mobile drug delivery devices such as the injector of Fig. 3, will typically have a wireless connection drawing a short term current in the range of at least 10mA to 100mA, which considerably higher than in the storage mode, even if no motor is active. To achieve optimal power consumption in active mode, the microprocessor 31 may therefore change the logic state of the power select line 30b and switch the power supply module 8 to active mode.

When changing to active mode, the second power supply circuitry 20 will switch on the FET transistor 23 and establish a connection between the second input power line 20b, which in turn is connected to the converter output power line 10b, and the control output power line 20c. As soon the control circuitry 30 starts drawing higher current, the voltage drop over the diode 21 makes sure that the voltage at the second input power line 20b. As the FET transistor 23 typically induces a much lower voltage drop than a diode, this means the control output power line 20c is more stable, particularly less affected by load changes. In active mode, the power supply module 8 will feed the control power line 20c from the converter output power line 10b and provide sufficiently high currents for all necessary activities, including drug delivery or wireless communication. The diode 21 blocks backfeeding the battery 4 from the converter output power line 10b.

A further key aspect of the present invention is the introduction of a start circuitry 40 as shown in the block diagram of Fig. 5. The start circuitry 40 is connected to the control circuitry 30 by a device activation signal line 40a and may get electric power from the control output power line 20c if needed. A multitude of electric lines or microprocessor pins may be used to realise the device activation signal line 40a, depending on the nature of the signal transmitted over this interface. The start circuitry 40 may actively or passively generate a signal on the device activation signal line 40a which can be used as a trigger to start an action such as a change of mode. An active signal can for example be a voltage applied to the device activation signal line 40a powered from the control output power line 20c, a passive signal can for example be an electric connection established between two microprocessor pins forming the device activation signal line 40a. In a simple embodiment, the start circuitry 40 includes an electromechanical switch such as a start button 1e, configured to generate a device activation signal by closing an electric contact between two microprocessor pins included in the device activation signal line 40a. More sophisticated embodiments may employ various sensors to detect a change of environmental conditions or a user action and trigger a change of mode of the drug delivery device 1 or the power supply module 8. As both the start circuitry 40 and the control circuitry 30 are at least to a minimal extent active in storage mode, and the control circuitry may always switch the power supply module 8 into a mode such as the active mode to provide sufficient electric current to itself, new possibilities for activation of the drug delivery device 1 arise. A device activation signal may be generated whenever an optic, acoustic, electric or thermal sensor allows the detection of an event related to preparing the drug delivery device 1 for use or for application to the body of a patient. One of many possible examples is the moment the user takes the device out of the packaging the device is shipped in and an optic sensor detects daylight. Or if an acceleration sensor detects a certain movement of the device. Or if a temperature sensor reports a temperature in the range of a human body, 36° C to 40° C. In the embodiment of the patch injector of Fig. 3, a preferred trigger for activation of the injector is to detect the moment the user removes the protective liner from the adhesive patch 1c or when the user attaches the patch injector to the body of the patient for application. To generate a device activation signal from such an event, the start circuitry 40 may have an electric sensor built into the adhesive patch 1c. In a preferred embodiment, the sensor may be a capacitive sensor. Removal of the liner and/or attachment to the body of the patient will change a capacity near the sensor, the capacitive sensor will detect the change, and the start circuitry 40 will either generate a trigger signal over the device activation signal line, or passively allow the control circuitry 30 to detect the change and trigger the change of mode from storage mode to active mode.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 1: Drug delivery device / patch pump / patch injector
- 1a: Housing
- 1b: Reservoir window
- 1c: Adhesive patch
- Id: Liner
- 1e: Start button
- If: Fill port
- 2: Reservoir
- 2a: Piston / Plunger
- 3: Fluid transport assembly
- 3a: Fluid input / Piercing needle
- 3b: Fluid output / Delivery needle
- 4: Battery with VBAT output voltage
- 5: Motor
- 6: Pump assembly
- 6a: Gear box assembly
- 6b: Threaded rod
- 6c: Segmented rod
- 6d: Plunger moving head
- 7: Printed Circuit Board PCB for the Power supply module and the electronic control unit
- 8: Power supply module
- 10: First power supply circuitry with VOUT output voltage
- 10a: Converter input power line
- 10b: Converter output power line
- 11: DCDC-Converter
- 20: Second power supply circuitry with VUC output voltage
- 20a: First input power line
- 20b: Second input power line
- 20c: Control output power line
- 21: Diode
- 22: Ideal Diode Component
- 23: FET Transistor
- 30: Control circuitry
- 30a: Converter output activation line DC on
- 30b: Power select line PWR_sel
- 31: Microprocessor
- 40: Device activation signal generator / Start circuitry
- 40a: Device activation signal line ACT

## Claims

1. A mobile or wearable drug delivery device (1) comprising
a battery (4);
an electric motor (5) for actuating a pump assembly (6) to convey a medical fluid from a reservoir (2) to a fluid output (3b);
an electronic control circuitry (30) comprising a microprocessor (31) configured to control the operation of the drug delivery device (1);
an electric power supply module (8) with
a first power supply circuitry (10) having a converter input power line (10a) connected to the battery (4), a DC-DC converter (11) and a converter output power line (10b) connected to the electric motor (5); and with
a second power supply circuitry (20) having a first input power line (20a) connected to the battery (4), a second input power line (20b) connected to the converter output power line (10b), and a control output power line (20c) connected to the control circuitry (30); configured to conduct electric current from the first input power line (20a) or the second input power line (20b) to the control output power line (20c);
***characterised by***
the control circuitry (30) comprises a power select line (30b) connected to the second power supply circuitry (20) configured to adopt at least two different logic states; and
the second power supply circuitry (20) is configured to switch on and off the electrical current from one of the input power lines (20a, 20b) to the control output power line (20c) according to the logic state of the power select line (30b); and
the control circuitry (30) is configured to switch the power supply module (8) from a storage mode, where the control circuitry (30) is substantially supplied with electric current from the first input power line (20a), to an active mode, where the control circuitry (30) is substantially supplied with electric current from the second input power line (20b), by switching the power select line (30b) from a first logic state to a second logic state.

2. A mobile or wearable drug delivery device (1) according to claim 1, whereby the second power supply circuitry (20) comprises a diode (21) in the electrical path from the first input power line (20a), and a FET transistor (23) in the electrical path from the second input power line (20b), whereby the gate input of the FET transistor (23) is connected to the power select line (30b).

3. A mobile or wearable drug delivery device according to claim 1 or 2, where the first and the second logic state of the power select line are logic states 0, 1 or Z such as defined for typical digital logic output states of the microprocessor.

4. A mobile or wearable drug delivery device according to claim 1 or 2, where the first and the second logic state of the power select line are two voltage levels with a difference of at least 0.1V in a typical range of analog output voltage levels of the microprocessor, for example 0V to 3V.

5. A mobile or wearable drug delivery device according to any preceding claim, whereby the drug delivery device is an infusion pump.

6. A mobile or wearable drug delivery device 1 according to any preceding claims, where the pump assembly (3) has a fluid input (3a) configured to be connected to the reservoir (2) and a fluid output (3b) configured to be connected to a patient.

7. A mobile or wearable drug delivery device (1) according to any preceding claim, further comprising a start circuitry (40) with a device activation signal line (40a), configured to generate a device activation signal for the control circuitry (30) as a trigger signal to change the mode of the power supply module (8) from storage mode to active mode.

8. A mobile or wearable drug delivery device (1) according to claim 7, where the start circuitry (40) includes a start button (1e).

9. A mobile or wearable drug delivery device (1) according to claims 7 or 8, where the start circuitry (40) includes an optic, acoustic, electric or thermal sensor to detect if the device is being prepared for use or applied to the body of a patient.

10. A mobile or wearable drug delivery device according to any preceding claim, whereby the drug delivery device (1) is a patch injector or patch pump with an adhesive patch (1c).

11. A patch injector or patch pump according to claim (10), where the start circuitry (40) has an electric sensor to detect if the protective liner (1d) is removed from the adhesive patch (1c).

12. A patch injector or patch pump according to claims 9 to 11, where the sensor is a capacitive sensor.
